(19) Europäisches Patentamt
European Patent Office
Office européen des brevets

(11) **EP 1 187 854 B1**

(12) **FASCICULE DE BREVET EUROPEEN**

(45) Date de publication et mention de la délivrance du brevet:
**10.11.2004 Bulletin 2004/46**

(21) Numéro de dépôt: **01919576.7**

(22) Date de dépôt: **27.03.2001**

(51) Int Cl.[7]: **C08B 37/16**, B01J 20/24, C02F 1/28, A61K 31/715

(86) Numéro de dépôt international:
**PCT/FR2001/000923**

(87) Numéro de publication internationale:
**WO 2001/072849 (04.10.2001 Gazette 2001/40)**

(54) **DERIVES DE PER(3,6-ANHYDRO) CYCLODEXTRINES, LEUR PREPARATION ET LEUR UTILISATION POUR SEPARER DES IONS**

DERIVATE VON PER(3,6-ANHYDRO) CYCLODEXTRINEN, DEREN HERSTELLUNG UND VERWENDUNG ZUR ABTRENNUNG VON IONEN

PER(3,6-ANHYDRO)CYCLODEXTRIN DERIVATIVES, PREPARATION AND USE THEREOF FOR SEPARATING IONS

(84) Etats contractants désignés:
**AT BE CH CY DE DK ES FI FR GB GR IE IT LI LU MC NL PT SE TR**

(30) Priorité: **28.03.2000 FR 0003899**

(43) Date de publication de la demande:
**20.03.2002 Bulletin 2002/12**

(73) Titulaires:
- **COMMISSARIAT A L'ENERGIE ATOMIQUE 75752 Paris Cédex 15 (FR)**
- **CENTRE NATIONAL DE LA RECHERCHE SCIENTIFIQUE (CNRS) 75794 Paris Cedex 16 (FR)**

(72) Inventeurs:
- **GADELLE, Andrée F-38330 Montbonnot (FR)**
- **FAUVELLE, Florence F-38000 Grenoble (FR)**
- **DEBOUZY, Jean-Claude F-38660 La Terrasse (FR)**

(74) Mandataire: **Audier, Philippe André et al Brevalex, 3, rue du Docteur Lancereaux 75008 Paris (FR)**

(56) Documents cités:
**EP-A- 0 787 744** **WO-A-98/56829**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet de nouveaux dérivés de per(3,6-anhydro)cyclodextrines, utilisables en particulier pour fixer et séparer des ions tels que les ions de cobalt, de lanthanides et d'uranyle.

Elle peut être appliquée en particulier dans le domaine de la décontamination de l'environnement en ces ions polluants, ainsi que pour la décontamination humaine.

## Etat de la technique antérieure

Les cyclodextrines ou cyclomaltooligosaccharides sont des composés d'origine naturelle formés par l'enchaînement d'unités glucose liés en α-(1,4).

De nombreux travaux ont montré que ces composés pouvaient former des complexes d'inclusion avec des molécules hydrophobes permettant ainsi leur solubilisation dans des milieux aqueux. De nombreuses applications ont été proposées pour tirer profit de ce phénomène, en particulier dans le domaine pharmaceutique, comme il est décrit par D. Duchêne "Pharmaceutical application of cyclodextrins" dans "Cyclodextrins and their industrial uses". D. Duchêne Ed., Editions de Santé, Paris, 1987, pages 213-257 [1].

Des spécialités pharmaceutiques ont déjà été commercialisées au Japon, en Italie et plus récemment en France, sous forme de complexes dans les cyclodextrines. En France, le premier principe actif mis sur le marché sous la forme d'un complexe d'inclusion dans une cyclodextrine est le piroxicam, anti-inflammatoire commercialisé par Pierre Fabre Médicament, sous le nom de BREXIN®. Parmi les très nombreux dérivés modifiés de ces cyclodextrines, ceux pour lesquels la cavité est retournée sur elle-même présentent des propriétés intéressantes même si leur capacité à inclure des molécules organiques est perdue ou très limitée. Des composés de ce type sont les per(3,6-anhydro) cyclodextrines.

La synthèse de ces peranhydrocyclodextrines a été décrite dès 1991 dans le document [2] : Gadelle A. et Defaye J., Angew. Chem. Int. Ed. Engl., (1991), 30, pages 78-79 ; et le document [3] : Ashton P.R., Ellwood P., Staton I. and Stoddart J.F., Angew . Chem. Int. ed. Engl., (1991) 30, pages 80-81), et il a été montré que ces dérivés présentent des solubilités intéressantes aussi bien dans l'eau que dans les solvants organiques. Quelques études ultérieures (document [4] : Yamamura H. and Fujita K. Chem. Pharm. Bull., (1991) 39, pages 2505-2508 ; document [5] : Yamamura H., Ezuka T., Kawase Y., Kawai M., Butsugan Y. and Fujita K., J. Chem. Soc., Chem. Com., (1993), pages 636-637 ; et document [6] : Yamamura H. Nagaoka H., Kawai M. and Butsugan Y., Tetrahedron Lett. (1995) 36, pages 1093-1094) ont de plus montré que ces dérivés peranhydro pouvaient complexer des ions alcalins avec une sélectivité non négligeable.

Le document FR-A-2 744 124 [7] et le document FR-A-2 764 525 [8] illustrent d'autres dérivés de per(3,6-anhydro)cyclodextrines substituées en position-2, utiles pour la séparation de différents ions, notamment le potassium et le césium dans le cas du document [7] grâce à la présence du substituant acétyle, ou le plomb dans le cas du document [8] grâce à la présence d'un substituant méthyle.

Cependant, les dérivés décrits dans ces documents ne permettent pas d'assurer une séparation satisfaisante par complexation des ions de cobalt, d'uranyle et de lanthanides tels que le dysprosium, qui polluent l'environnement.

De plus, les ions de lanthanides sont toxiques pour les êtres vivants en troublant les échanges ioniques du calcium et du sodium. Ainsi, le lanthane qui est de même taille que le calcium mais non de même valence pertube les échanges comme il est décrit par Evans CH, « Interactions of Lanthanides with Tissues, Cells and Cellular Organelles » dans Biochemistry of the Lanthanides, Evans C. H. Ad., Plenum Press, New York, 1990, pp. 211-283 [9].

## Exposé de l'invention

La présente invention a précisément pour objet de nouveaux dérivés de peranhydrocyclodextrines dans lesquels le substituant en position-2 a été choisi pour leur conférer des propriétés de complexation des ions polluants tels que $Co^{2+}$, $UO_2^{2+}$ et les ions de lanthanides comme $Dy^{3+}$ et $Eu^{3+}$.

Selon l'invention le dérivé de per(3,6-anhydro)cyclodextrine répond à l'une des formules suivantes :

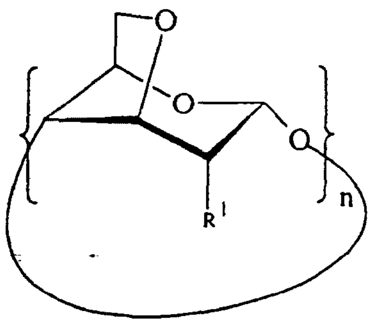 (I) et 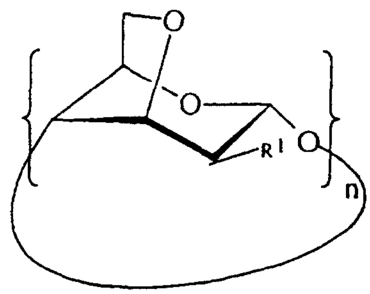 (II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH et $R^2$, dans lesquelles $R^2$ et $R^3$ qui peuvent être identiques ou différents représentent un groupe hydrocarboné, aliphatique ou aromatique, saturé ou insaturé, pouvant comporter un ou plusieurs hétéroatomes choisis parmi O, S et N, et n est égal à 6, 7 ou 8.

Dans le dérivé de cyclodextrine de formule (I) ou (II), les groupes hydrocarbonés aliphatiques ou aromatiques, susceptibles d'être utilisés pour $R^2$ et $R^3$ peuvent être de divers types. Ils sont constitués par une chaîne carbonée dans laquelle certains atomes de carbone peuvent être remplacés par un ou plusieurs hétéroatomes tels que 0, S et N, et ils peuvent comporter une ou plusieurs insaturations éthyléniques ou acétyléniques. Par ailleurs, le groupe hydrocarboné peut comporter différents substituants, en particulier des groupes fonctionnels ou des atomes d'halogènes. Les groupes hydrocarbonés aromatiques peuvent être constitués par le groupe phényle et le groupe tosyle, éventuellement substitués, par exemple par des groupes alkyle de 1 à 20 atomes de carbone.

$R^2$ et $R^3$ peuvent en particulier représenter un groupe alkyle linéaire ou ramifié de 1 à 20 atomes de carbone.

Selon un mode de réalisation préféré de l'invention, le dérivé de per(3,6-anhydro)cyclodextrine est un dérivé d'$\alpha$-cyclodextrine, c'est-à-dire que dans les formules (I) et (II) données ci-dessus, n est égal à 6.

De préférence encore, le dérivé utilisé répond à la formule (I) dans laquelle tous les $R^1$ représentent le groupe $-OCH_2COOH$ et n est égal à 6.

Les dérivés de cyclodextrine de l'invention peuvent être préparés par différents procédés.

Lorsque le dérivé de cyclodextrine répond à la formule (I) ou (II) donnée ci-dessus dans laquelle au moins l'un des $R^1$ représente le groupe $-OCH_2COOH$, les autres $R^1$ représentant OH ou un autre groupe et n étant égal à 6, 7 ou 8, ceux-ci peuvent être préparés par un procédé comprenant les étapes suivantes :

- 1) faire réagir une peranhydrocyclodextrine répondant à l'une des formules :

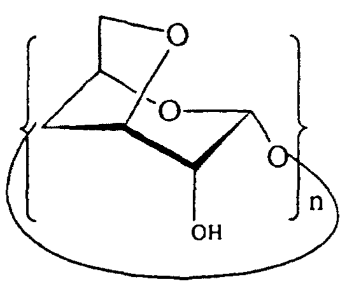 (III) ou 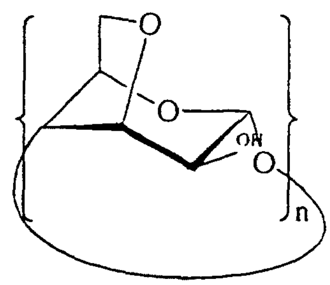 (IV)

dans lesquelles n est égal à 6, 7 ou 8, avec un hydrure de métal alcalin pour convertir le(s) groupe(s) OH en groupe (s) OM avec M représentant un métal alcalin ;

- 2) faire réagir en milieu alcalin la peranhydrocyclodextrine modifiée obtenue en 1) avec un halogénure de fcrmule $XCH_2COOR^4$ dans laquelle X représente un atome d'halogène tel que Cl, et $R^4$ représente H, Si $(CH_3)_3$ ou un métal alcalin, en quantité telle que l'un au moins de(s) groupe(s) OM soit transformé en groupe $-CH_2COOR^4$ ;
- 3) faire réagir, dans le cas où tous les groupes OM n'ont pas été transformés en groupe $-OCH_2COOR^4$, les groupes OM restants avec un ou plusieurs réactifs pour les transformer en les groupes $R^1$ voulus différents de $-OCH_2COOH$ ; et
- 4) traiter le dérivé de peranhydrocyclodextrine obtenu en 3) avec un alcool, de l'eau ou un milieu légèrement acide

pour transformer le(s) groupe(s) $-OCH_2COOR^4$ en groupe $-OCH_2COOH$.

Pour effectuer l'étape 2), on utilise la quantité nécessaire de $XCH_2COOR^4$ pour modifier un ou plusieurs des groupe OH de la cyclodextrine.

Dans l'étape 4), lorsque $R^4$ représente M on transforme les groupes $-OCH_2COOR^4$ en $-OCH_2COOH$ par action d'un alcool tel que le méthanol. On peut aussi utiliser de l'eau mais la réaction sera plus violente.

Lorsque $R^4$ représente $Si(CH_3)_3$, on utilise un milieu légèrement acide pour régénérer la fonction acide.

Lorsque le dérivé de cyclodextrine répond à la formule (I) ou (II) donnée ci-dessus dans laquelle les autres $R^1$ représentent $OR^2$ avec $R^2$ ayant la signification donnée ci-dessus, on procède comme précédemment pour introduire le(s) groupe(s) $OCH_2COOM$, puis on fait réagir ensuite le dérivé avec un halogénure de formule $R^2X$ dans laquelle $R^2$ a la signification donnée ci-dessus et X est un atome d'halogène.

Lorsque le dérivé de cyclodextrine répond à la formule (I) ou (II) dans laquelle les autres $R^1$ représentent $OCOR^2$, on procède comme précédemment pour introduire tout d'abord les groupes $OCH_2COOM$, puis on fait réagir ensuite le dérivé obtenu avec un halogénure ou anhydride d'acide de formules $R^2COX$ ou $(R^2CO)_2O$ dans lesquelles $R^2$ a la signification donnée ci-dessus et X représente un atome d'halogène, pour remplacer les hydroxyles restants par $OCOR^2$.

Lorsque l'on veut préparer un dérivé de cyclodextrine dans lequel le(s) autre(s) $R^1$ représentent un atome d'halogène ou un groupe de formule SH, $SR^2$, $NH_2$, $NR^2R^3$, $CONR^2R^3$, $CONH_2$, CN, $COOR^2$, COOH, ou $R^2$, avec $R^2$ et $R^3$ ayant les significations données ci-dessus, et n est égal à 6, 7 ou 8, on peut effectuer les étapes suivantes en partant d'une peranhydrocyclodextrine partiellement modifiée, c'est-à-dire dans laquelle l'un au moins des $R^1$ représente $OCH_2COOH$ et les autres $R^1$ représentent OH, et en effectuant les étapes suivantes :

1) faire réagir cette peranhydrocyclodextrine avec un hydrure de métal alcalin pour convertir le(s) groupe(s) OH en groupe(s) OM avec M représentant un métal alcalin ;
2) faire réagir la peranhydrocyclodextrine modifiée obtenue en 1) avec un chlorure de formule $ClSO_2R^2$ avec $R^2$ ayant la signification donnée ci-dessus, pour obtenir le dérivé de formule (I) ou (II) dans laquelle l'un au moins des $R^1$ est un groupe de formule $OSO_2R^2$ ; et
3) faire réagir le dérivé obtenu dans la deuxième étape avec un ou plusieurs réactifs appropriés pour remplacer $OSO_2R^2$ par le groupe $R^1$ voulu.

Dans ce procédé on transforme tout d'abord la per(3,6-anhydro)cyclodextrine en alcoolate par action d'hydrure de métal alcalin, puis on convertit cet alcoolate en dérivé comportant un groupe partant de formule $OSO_2R^2$, que l'on fait réagir ensuite en une ou plusieurs étapes avec un ou plusieurs réactifs appropriés pour remplacer ce groupe partant par le groupe $R^1$ voulu.

Ainsi, dans le cas où $R^1$ doit représenter $NH_2$, on peut faire réagir $N_3M$ et le composé défini en 2). Le composé ainsi obtenu appelé azide peut subir une hydrogénation catalytique ou être traité en présence d'ammoniac $NH_3$, afin d'obtenir le produit où $R^1$ doit représenter $NH_2$.

Le produit où $R^1$ doit représenter $NHR^2$ ou $NR^2R^3$ est obtenu en faisant réagir le composé défini en 2) sur le composé $NH_2R^2$ ou $NHR^2R^3$.

Dans le cas où $R^1$ doit représenter SH ou $SR^2$, on peut faire réagir le composé défini en 2) avec un halogénure $X^-$, ce qui donne le composé avec ($R^1$ = X), que l'on fait ensuite réagir avec $HS^-$ ou $R^2S^-$ pour donner un composé où $R^1$ représentera SH ou $SR^2$.

Lorsque $R^1$ doit représenter un groupe hydrocarboné, on fait réagir avec $R^1_2LiCu$ ($R^1$ représente un groupe hydrocarboné) pour donner un composé final où $R^1$ représente alors un groupe hydrocarboné.

De même, le composé où $R^1$ représente un halogène peut réagir avec $CN^-$ pour donner un composé final où $R^1$ représentera CN.

De même, le composé où $R^1$ représente CN peut par hydrolyse ménagée donner un composé où $R^1$ représentera $CONH_2$. Le composé où $R^1$ représente CN peut par hydrolyse complète donner un composé où $R^1$ représentera COOH.

Le composé où $R^1$ représente COOH peut par estérification donner un composé où $R^1$ représentera $COOR^2$.

Le composé où $R^1$ représente COOH peut réagir sur $NHR^2R^3$ ou $NH_2R^2$ en présence de DCC (dicyclohexylcarbodiimide) pour donner un composé où $R^1$ représentera $NR^2R^3$ ou $NH_2R^2$.

Les dérivés de per(3,6-anhydro)cyclodextrine de l'invention peuvent être utilisés en particulier pour la fixation ou la séparation d'ions.

Aussi, l'invention a également pour objet un procédé de fixation ou de séparation d'ions consistant à mettre en contact un milieu contenant lesdits ions avec un dérivé de per (3,6-anhydro)cyclodextrine répondant à l'une des formules suivantes :

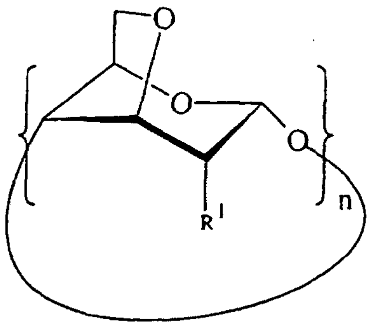

(I) et

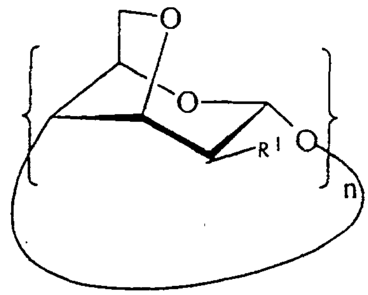

(II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONHR^2$, $CONR^2R^3$, $CONH_2$, CN, $COOR^2$, COOH et $R^2$, dans lesquelles $R^2$ et $R^3$ qui peuvent être identiques ou différents, représentent un groupe hydrocarboné, aliphatique ou aromatique, saturé ou insaturé, pouvant comporter un ou plusieurs hétéroatomes choisis parmi O, S et N, et n est égal à 6, 7 ou 8,
pour fixer lesdits ions sous forme de complexe avec le dérivé de per(3,6-anhydro)cyclodextrine et les séparer dudit milieu.

Les ions susceptibles d'être fixées ou séparés par le procédé de l'invention peuvent être de divers types ; il peut s'agir par exemple d'ions d'actinides, par exemple d'uranyle, de lanthanides ou de métaux polluants tels que le cobalt.

Le procédé de l'invention s'applique en particulier à la séparation et à la fixation du cobalt et des ions de lanthanides sous forme de complexe.

En effet, le cobalt, les lanthanides et ses dérivés polluent l'environnement et sont toxiques aussi bien chez l'animal que chez l'homme. Les principaux effets toxiques affectent le développement neurologique et le fonctionnement du système nerveux. Il est donc nécessaire de séparer et d'éliminer ces ions de l'environnement et de le stocker de manière sûre.

Par ailleurs, des produits qui permettraient d'assurer la décontamination en cobalt et en lanthanides des êtres vivants en empêchant leur action sur le système nerveux et sur d'autres organes, seraient d'un grand intérêt pour résoudre ces problèmes.

Selon l'invention, on a trouvé que les dérivés des per(3,6-anhydro)cyclodextrines répondant aux formules (I) et (II) données ci-dessus, présentaient une spécificité élevée pour le cobalt et les lanthanides, et étaient capables de complexer ceux-ci avec des rendements élevés pouvant atteindre 100 %, même en présence d'autres ions tels que les ions sodium.

De cette façon, on peut séparer le cobalt et les lanthanides du milieu environnant sous la forme de complexe.

Aussi, l'invention a également pour objet les complexes d'un métal choisi parmi Dy, Eu, Lu, La et Co et de dérivés de per(3,6-anhydro)cyclodextrines de formule (I) ou (II) décrits ci-dessus.

Pour mettre en oeuvre le procédé de séparation d'ions de l'invention, on peut utiliser le dérivé de per(3,6-anhydro)cyclodextrine de formule (I) ou (II) sous forme de solution aqueuse ou de solution organique.

Lorsque le milieu contenant les ions à séparer ou à fixer est une solution aqueuse, on peut dissoudre le dérivé de cyclodextrine dans un solvant organique immiscible avec la solution aqueuse, par exemple dans du chloroforme, pour former le complexe dans la solution organique et le séparer facilement de la solution aqueuse.

On peut aussi utiliser le dérivé de cyclodextrine en solution aqueuse, notamment pour assurer la décontamination des êtres vivants.

En effet, on sait que les dérivés de cyclodextrines de formule (I) ou (II) sont des composés biocompatibles. Ils peuvent donc être administrés à l'homme ou à l'animal pour assurer la fixation du cobalt et des lanthanides sous forme de complexe et éviter ainsi leur interaction avec les organes du corps humain ou animal.

Aussi, l'invention a également pour objet une composition pharmaceutique pour la décontamination en lanthanides et en cobalt d'un être vivant, caractérisée en ce qu'elle comprend un dérivé de per(3,6-anhydro)cyclodextrine répondant à l'une des formules (I) et (II), décrit ci-dessus.

De préférence, le dérivé de per(3,6-anhydro)cyclodextrine utilisé dans cette composition répond à la formule (I) dans laquelle tous les $R^1$ représentent le groupe $-OCH_2COOH$ et n est égal à 6.

Cette composition peut être administrée par voie orale ou par injection.

Les solutions aqueuses peuvent comprendre jusqu'à 0,08 mol/l de dérivé de formule (I).

Les quantités administrées dépendront du taux de contamination et du poids du patient.

Les dérivés de cyclodextrine de l'invention présentent de nombreux avantages. En particulier lorsqu'ils sont persubstitués, c'est-à-dire lorsque tous les $R^1$ sont différents du groupe OH, on a des dérivés qui présentent une bonne solubilité dans les solvants organiques tels que le chloroforme, l'acétone, le tétrahydrofurane etc. Cette solubilité est intéressante pour leur utilisation dans la séparation ionique car elle permet de réaliser la séparation par des procédés d'échanges liquide-liquide qui sont bien connus dans la technique.

Par ailleurs, la possibilité d'introduire un ou plusieurs groupes chimiques particuliers permet de construire sur mesure des agents complexants pour des ions très divers. Cette facilité est de plus amplifiée par le fait que les trois cyclodextrines naturelles qui peuvent être utilisées comme matière de base, ont des diamètre de cavité différents qui peuvent apporter une sélection supplémentaire en rapport avec la taille des ions à séparer.

Les produits de départ de formules (III) ou (IV) utilisés dans l'invention peuvent être préparés par des procédés classiques tels que ceux décrits dans les documents [2] et [3] précités de Gadelle A. et al. et de Ashthon P. R. et al.

D'autres caractéristiques et avantages de l'invention apparaîtront mieux à la lecture des exemples qui suivent, donnés à titre illustratif et non limitatif en référence aux dessins annexés.

## Brève description des dessins

La figure 1 illustre les spectres de résonance magnétiques nucléaire (RMN) du proton du dérivé de l'exemple 1 seul (CD) en solution à 1 mmol/L, ou en présence de 4 mmol/L de $Lu^{3+}$, $La^{3+}$,$Dy^{3+}$, $Eu^{3+}$ et $Co^{2+}$.

La figure 2 illustre les spectres de RMN du proton du dérivé de l'exemple 1 en solution à 1 mmol/L, en présence d'éthylène diamine tétracétate et de 4 mmol/L de $Dy^{3+}$, $Eu^{3+}$ et $Co^{2+}$.

La figure 3 illustre les spectres de RMN du proton du dérivé de l'exemple 1 seul (CD) et en présence des cations physiologiques $Na^+$, $K^+$ et $Ca^{2+}$.

## Exposé détaillé des modes de réalisation

### Exemple 1 : Préparation de l'hexakis (3,6-anhydro-2-0-carboxyméthyl)cyclomaltohexaose.

Ce composé répond à la formule (I) donnée ci-dessus dans laquelle tous les $R^1$ représentent $OCH_2COOH$ et n est égal à 6.

On pèse 1 g (1,15 mmol) d'hexakis (3,6-anhydro)- cyclomaltohexaose séché sous vide pendant 2 heures à 120°C, et on ajoute 10 mL de diméthylsulfoxyde DMSO anhydre et 10 mL d'une solution de DMSO ayant réagi avec de l'hydrure de sodium (solution 2N d'hydrure de sodium dans le DMSO). La solution est maintenue sous agitation et sous atmosphère d'argon à température ambiante pendant 3 heures. Une solution gris bleue est obtenue. On ajoute alors du monochloroacétate de sodium (1,6 g, 14 mmol). La solution est laissée à température ambiante pendant 24 heures, puis le courant d'argon est supprimé. La solution est alors traitée par 10 mL d'alcool méthylique, amenée soigneusement à sec, reprise par de l'acétone et filtrée. La poudre obtenue dissoute dans l'eau est neutralisée par de l'acide chlorhydrique (solution 1N), et dialysée contre l'eau pendant 24 heures (Spectra/Port®CE Sterile DispoDialysers® -membrane d'ester de cellulose-MWCO 500). La réaction est quantitative. Le dialysat est lyophilisé et caractérisé par la résonnance magnétique du proton et du carbone.

La figure 1 illustre le spectre de résonance magnétique nucléaire du proton concernant ce produit (CD).

On l'utilise ensuite tel quel pour les complexations mises en oeuvre dans les exemples qui suivent.

### Exemple 2 : Préparation de complexes de l'hexakis (3,6-anhydro-2-0-carboxyméthyl) cyclomatéhexaose.

Chaque complexe est préparé en ajoutant à 500 µL d'une solution aqueuse contenant 1 mmol/L du produit de l'exemple 1, 4 mmol/L du cation testé, et en utilisant les cations suivants : $Lu^{3+}$, $La^{3+}$,$Dy^{3+}$, $Eu^{3+}$ et $Co^{2+}$ .

On caractérise les complexes par résonance magnétique nucléaire du proton. Les spectres obtenus sont représentés sur la figure 1 pour $Lu^{3+}$, pour $La^{3+}$, pour $Dy^{3+}$, pour $Eu^{3+}$ et pour $Co^{2+}$ .

Si l'on compare ces spectres avec le spectre du produit de l'exemple 1 seul (CD), on remarque que le spectre est très nettement modifié par l'ajout des cations testés.

Dans le cas des ions $Dy^{3+}$, $Eu^{3+}$ et $Co^{2+}$, on a une interaction très forte entre ces ions et le peracide. En effet, le spectre du dérivé de per(3,6-anhydro)cyclodextrine a totalement disparu, indiquant une immobilisation totale des protons impliqués dans l'interaction.

Dans le cas du lanthane, les protons H6 et H6' situés sur le pont anhydro sont encore observables.

Le cas du lutétium est plus complexe : le spectre de la cyclodextrine devient très compliqué avec l'apparition d'une multitude de résonances non attribuables directement. Il est probable que plusieurs complexes de stoechiométries différentes coexistent en solution.

**Exemple 3**

Dans cet exemple, on réalise des expériences de compétition entre le dérivé de l'exemple 1 et l'éthylènediaminetétracétate (EDTA) pour la complexation des ions $Dy^{3+}$, $Eu^{3+}$ et $Co^{2+}$, afin d'avoir une idée de la force des complexes préparés dans l'exemple 2.

Dans ce but, on utilise 50 μL de solution aqueuse contenant 1 mmol/L du dérivé de l'exemple 1 à laquelle on ajoute 4 mmol/L du cation testé et de l'EDTA. On caractérise les produits par RMN du proton.

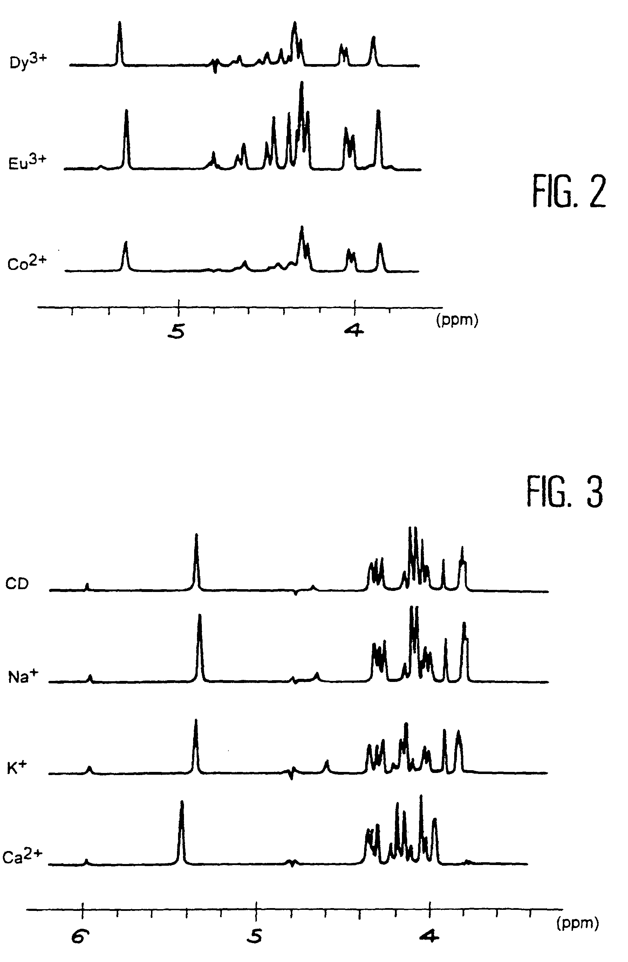

Les spectres obtenus sont représentés sur la figure 2. Sur cette figure, on remarque que l'addition d'EDTA permet dans tous les cas de retrouver un spectre partiel de cyclodextrine. Cependant malgré l'addition d'un large excès d'EDTA par rapport à la cyclodextrine, le spectre de cyclodextrine retrouvé n'est pas total. Ceci démontre que le dérivé de cyclodextrine complexe les cations plus fortement que l'EDTA.

**Exemple 4**

Dans cet exemple, on teste les propriétés de complexation du dérivé de l'exemple 1 vis-à-vis des cations physiologiques : calcium, sodium et potassium qui sont en fait tous les cations nécessaires au développement des êtres vivants.

En effet, pour une application en décontamination humaine, il convient de s'assurer que le dérivé ne complexe pas les cations physiologiques.

On suit le même mode opératoire que dans l'exemple 2 et on caractérise les produits par RMN du proton.

La figure 3 illustre les résultats obtenus respectivement avec $Na^+$, $K^+$ et $Ca^{2+}$.

Sur cette figure, le spectre (CD) correspond au dérivé de l'exemple 1 seul.

On remarque ainsi que les raies du dérivé de l'exemple 1 sont très peu affectées par la présence des cations physiologiques, par comparaison avec les spectres de la figure 1.

**LISTE DES DOCUMENTS CITES**

[1]: D. Duchêne "Pharmaceutical application of cyclodextrins" dans "Cyclodextrins and their industrial uses". D. Duchêne Ed., Editions de Santé, Paris, 1987. pages 213-257.

[2]: Gadelle A. et Defaye J., Angew. Chem. Int. Ed. Engl., 1991, 30, pages 79-79.

[3]: Ashton P.R., Ellwood P., Staton I and Stoddart J.F., Angew. Chem. Int. ed. Engl., 1991, 30, pages 80-81.

[4]: Yamamura H. and Fujita K., Chem. Pharm. Bull., 1991, 39, pages 2505-2508.

[5] : Yamamura H., Esuka T., Kawase Y., Kawai M.. Butsugan Y. and Fujita K., J. Chem. Soc., Chem. Commun., 1993, pages 636-637.

[6]: Yamamura H., Nagaoka H., Kawai M and Butsugan Y., Tetrahedron Lett. , 1995, 3b, pages 1093-1094.

[7] FR-A-2 744 124

[8] FR-A-2 764 525

[9] Evans CH, « Interactions of Lanthanides with Tissues, Cells and Cellular Organelles » dans Biochemistry of the Lanthanides. Evans C. H. Ad., Plenum Press, New York, 1990, pages 211-283.

**Revendications**

1. Dérivé de per(3,6-anhydro)cyclodextrine répondant à l'une des formules suivantes :

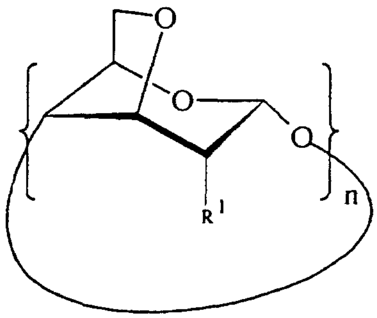

(I) et

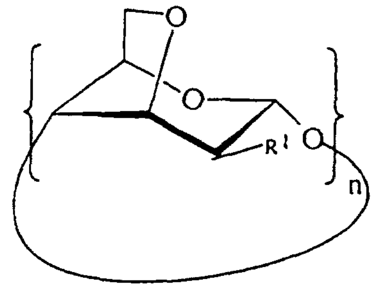

(II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH et $R^2$, dans lesquelles $R^2$ et $R^3$ qui peuvent être identiques ou différents, représentent un groupe hydrocarboné, aliphatique ou aromatique, saturé ou insaturé, pouvant comporter un ou plusieurs hétéroatomes choisis parmi O, S et N, et n est égal à 6, 7 ou 8.

**2.** Dérivé de per (3,6-anhydro)cyclodextrine selon la revendication 1, dans lequel tous les $R^1$ représentent le groupe $-OCH_2COOH$, et n est égal à 6.

**3.** Procédé de préparation d'un dérivé de per(3,6-anhydro)cyclodextrine répondant à l'une des formule (I) et (II) :

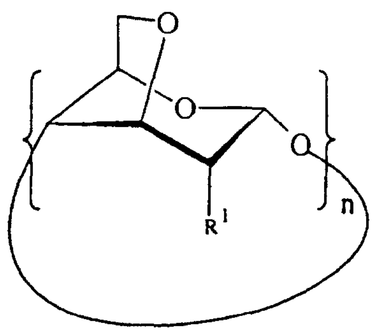

(I) et

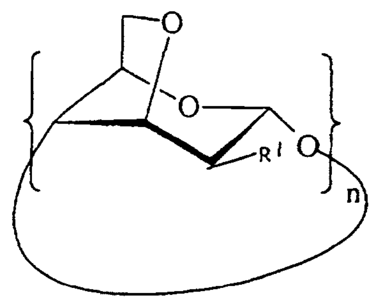

(II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH et $R^2$, dans lesquelles $R^2$ et $R^3$ qui peuvent être identiques ou différents, représentent un groupe hydrocarboné, aliphatique ou aromatique, saturé ou insaturé, pouvant comporter un ou plusieurs hétéroatomes choisis parmi 0, S et N, et n est égal à 6, 7 ou 8, qui comprend les étapes suivantes :

- 1) faire réagir une peranhydrocyclodextrine de formule :

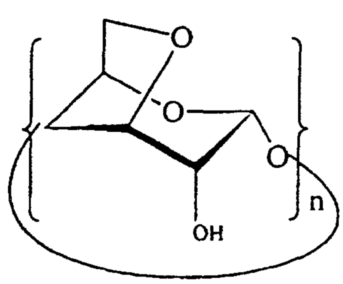

(III) ou

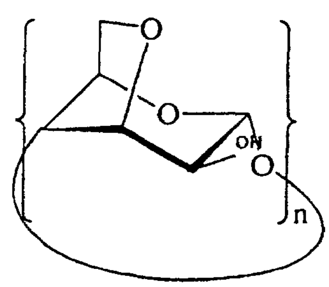

(IV)

dans lesquelles n est égal à 6, 7 ou 8, avec un hydrure de métal alcalin pour convertir le(s) groupe(s) OH en groupe(s) OM avec M représentant un métal alcalin ;

- 2) faire réagir en milieu alcalin la peranhydrocyclodextrine modifiée obtenue en 1) avec un halogénure de formule $XCH_2COOR^4$ dans laquelle X représente un atome d'halogène et $R^4$ représente H, Si $(CH_3)_3$ ou un métal alcalin, en quantité telle que l'un au moins de(s) groupe(s) OM soit transformé en groupe $-CH_2COOR^4$ ;
- 3) faire réagir, dans le cas où tous les groupes OM n'ont pas été transformés en groupe $-OCH_2COOR^4$, les groupes OM restants avec un ou plusieurs réactifs pour les transformer en les groupes $R^1$ voulus différents de $-OCH_2COOH$ ; et
- 4) traiter le dérivé de peranhydrocyclodextrine obtenu en 3) avec un alcool, un milieu légèrement acide ou de l'eau pour transformer le(s) groupe(s) $-OCH_2COOR^4$ en groupe $-OCH_2COOH$.

**4.** Procédé de fixation ou de séparation d'ions consistant à mettre en contact un milieu contenant lesdits ions avec un dérivé de per (3,6-anhydro)cyclodextrine répondant à l'une des formules suivantes :

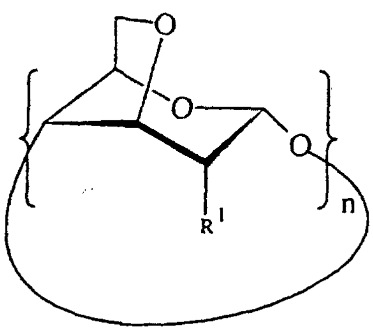
(I) et
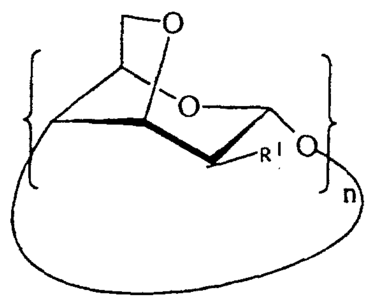
(II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONHR^2$, $CONR^2R^3$, $CONH_2$, CN, $COOR^2$, COOH et $R^2$, dans lesquelles $R^2$ et $R^3$ qui peuvent être identiques ou différents, représentent un groupe hydrocarboné, aliphatique ou aromatique, saturé ou insaturé, pouvant comporter un ou plusieurs hétéroatomes choisis parmi 0, S et N, et n est égal à 6, 7 ou 8,
pour fixer lesdits ions sous forme de complexe avec le dérivé de per(3,6-anhydro)cyclodextrine et les séparer dudit milieu.

**5.** Procédé selon la revendication 4, dans lequel lesdits ions sont des ions de cobalt, de lanthanides et/ou d'uranyle.

**6.** Procédé selon la revendication 4, dans lequel lesdits ions sont des ions de cobalt, de dysprosium et/ou d'europium.

**7.** Procédé selon l'une quelconque des revendications 4 à 6, dans lequel le dérivé de per(3,6-anhydro)cyclodextrine répond à la formule (I) dans laquelle tous les $R^1$ représentent le groupe $-OCH_2COOH$ et n est égal à 6.

**8.** Procédé selon l'une quelconque des revendications 4 à 7, dans lequel ledit milieu étant une solution aqueuse, le dérivé de per(3,6-anhydro)cyclodextrine est dissous dans un solvant organique immiscible avec la solution aqueuse.

**9.** Composition pharmaceutique pour la décontamination en lanthanides et en cobalt d'un être vivant, **caractérisée en ce qu'**elle comprend un dérivé de per(3,6-anhydro)cyclodextrine répondant à l'une des formules suivantes :

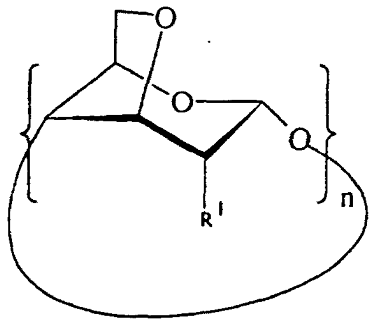 (I) et 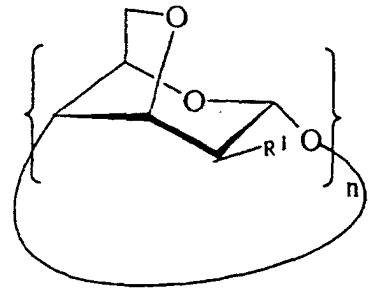 (II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH et $R^2$, dans lesquelles $R^2$ et $R^3$ qui peuvent être identiques ou différents, représentent un groupe hydrocarboné, aliphatique ou aromatique, saturé ou insaturé, pouvant comporter un ou plusieurs hétéroatomes choisis parmi O, S et N, et n est égal à 6, 7 ou 8.

**10.** Composition pharmaceutique selon la revendication 9, dans laquelle le dérivé de per(3,6-anhydro)cyclodextrine répond à la formule (I) dans laquelle tous les $R^1$ représentent le groupe $-OCH_2COOH$ et n est égal à 6.

**11.** Complexe d'un métal choisi parmi Dy, Eu, Lu, La et Co et d'un dérivé de per (3,6-anhydro)cyclodextrine répondant à l'une des formules suivantes :

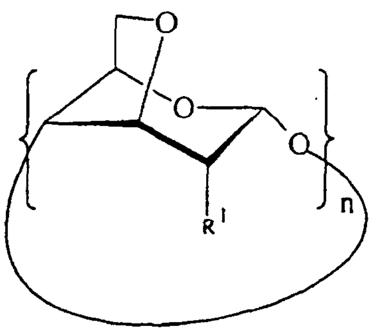 (I) et 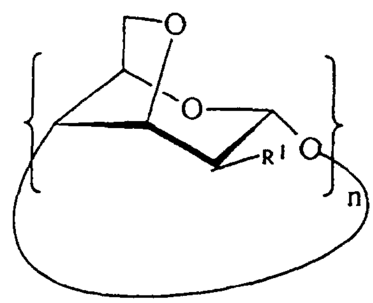 (II)

dans lesquelles l'un au moins des $R^1$ représente le groupe $-OCH_2COOH$ et les autres $R^1$ qui peuvent être identiques ou différents, représentent un groupe répondant à l'une des formules : OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH et $R^2$, dans lesquelles $R^2$ et $R^3$ qui peuvent identiques ou différents représentent un groupe hydrocarboné, aliphatique ou aromatique, saturé ou insaturé, pouvant comporter un ou plusieurs hétéroatomes choisis parmi O, S et N, et n est égal à 6, 7 ou 8.

**12.** Complexe selon la revendication 11, dans lequel le dérivé de per(3,6-anhydro) cyclodextrine répond à la formule (I) dans laquelle tous les $R^1$ représentent le groupe $-OCH_2COOH$ et n est égal à 6.

## Patentansprüche

**1.** Per(3, 6-anhydro)-cyclodextrin-Derivat, das einer der folgenden Formeln entspricht:

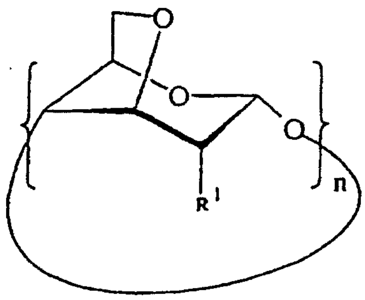 (I) und 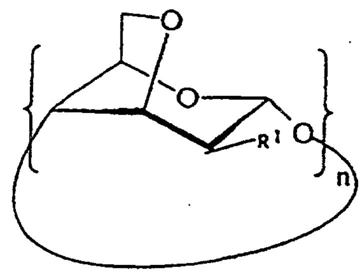 (II)

worin bedeuten:

mindestens einer der Reste $R^1$ die -$OCH_2COOH$-Gruppe und die übrigen Reste $R^1$, die gleich oder verschieden sein können, eine Gruppe mit einer der folgenden Formeln: OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH und $R^2$, worin $R^2$ und $R^3$, die gleich oder verschieden sein können, eine aliphatische oder aromatische, gesättigte oder ungesättigte Kohlenwasserstoffgruppe darstellen, die ein oder mehrere Heteroatome, ausgewählt aus der Gruppe O, S und N, enthalten kann, und

n die ganze Zahl 6, 7 oder 8.

**2.** Per(3, 6-anhydro)-cyclodextrin-Derivat nach Anspruch 1, bei dem alle Reste $R^1$ die Gruppe -$OCH_2COOH$ darstellen und n für die Zahl 6 steht.

**3.** Verfahren zur Herstellung eines Per(3, 6-anhydro)-cyclodextrin-Derivats, das einer der Formeln (I) und (II) entspricht:

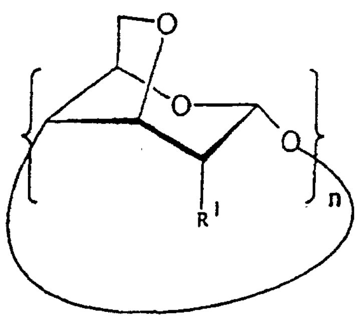 (I) und 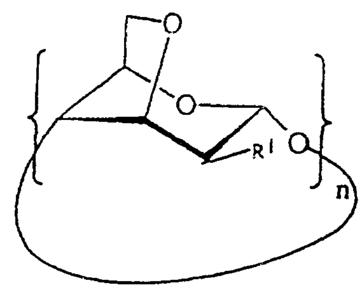 (II)

worin bedeuten:

mindestens einer der Rest $R^1$ die Gruppe -$OCH_2COOH$ und die übrigen Reste $R^1$, die gleich oder verschieden sein können, eine Gruppe mit einer der folgenden Formeln: OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH und $R^2$, worin $R^2$ und $R^3$, die gleich oder verschieden sein können, eine aliphatische oder aromatische, gesättigte oder ungesättigte Kohlenwasserstoffgruppe darstellen, die ein oder mehrere Heteroatome, ausgewählt aus der Gruppe O, S und N, enthalten kann, und

n die Zahl 6, 7 oder 8,

wobei das Verfahren die folgenden Stufen umfasst:

- (1) Reagierenlassen eines Peranhydrocyclodextrins der Formel:

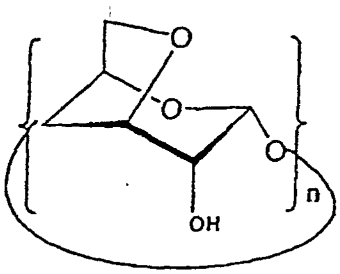 (III) oder 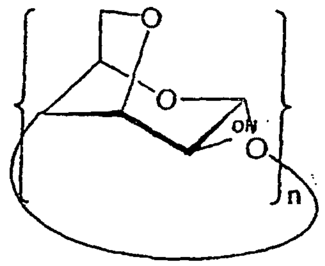 (IV)

worin n steht für die Zahl 6, 7 oder 8, mit einem Alkalimetallhydrid, um die OH-Gruppe(n) in eine oder mehrere OM-Gruppen, worin M für ein Alkalimetall steht, zu überführen;

- (2) Reagierenlassen des in der Stufe (1) erhaltenen modifizierten Peranhydrocyclodextrins in alkalischem Medium mit einem Halogenid der Formel $XCH_2COOR^4$, worin X steht für ein Halogenatom und $R^4$ steht für H, Si $(CH_3)_3$ oder ein Alkalimetall, in einer solchen Menge, dass mindestens eine der OM-Gruppen in eine $-CH_2COOR^4$-Gruppe überführt wird;
- (3) Reagierenlassen, für den Fall, dass nicht alle der OM-Gruppen in eine $-OCH_2COOR^4$-Gruppe überführt worden sind, der restlichen OM-Gruppen mit einem oder mehreren Reagentien, um sie in die gewünschten, von $-OCH_2COOH$ verschiedenen $R^1$-Gruppen zu überführen; und
- (4) Behandeln des in der Stufe (3) erhaltenen Peranhydrocyclodextrin-Derivats mit einem Alkohol in einem leicht sauren Medium oder in Wasser, um die $-OCH_2COOR^4$-Gruppe(n) in $-OCH_2COOH$-Gruppe(n) zu überführen.

**4.** Verfahren zum Fixieren oder Abtrennen von Ionen, das darin besteht, dass man ein Medium, das die genannten Ionen enthält, mit einem Per(3,6-anhydro)-cyclodextrin-Derivat, das einer der folgenden Formeln entspricht, in Kontakt bringt:

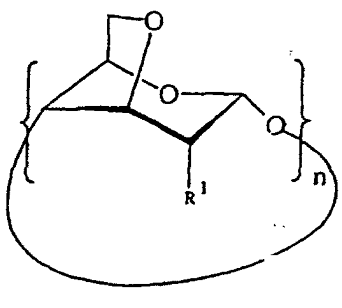 (I) und 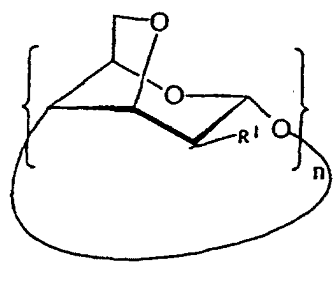 (II)

worin bedeuten:

mindestens einer der Reste $R^1$ die Gruppe $-OCH_2COOH$ und die übrigen Reste $R^1$, die gleich oder verschieden sein können, eine Gruppe mit einer der Formeln: OH, $OR^2$, SH, $SR^2$, $OCOR^2$, $NH_2$, $NHR^2$, $NR^2R^3$, $CONHR^2$, $CONR^2R^3$, $CONH_2$, CN, $COOR^2$, COOH und $R^2$, worin $R^2$ und $R^3$, die gleich oder verschieden sein können, eine aliphatische oder aromatische, gesättigte oder ungesättigte Kohlenwasserstoffgruppe darstellen, die ein oder mehrere Heteroatome, ausgewählt aus der Gruppe O, S und N, enthalten kann, und

n die Zahl 6, 7 oder 8,

um die genannten Ionen in Form eines Komplexes mit dem Per(3,6-anhydro)-cyclodextrin-Derivat zu fixieren und sie aus dem Medium abzutrennen.

**5.** Verfahren nach Anspruch 4, in dem die genannten Ionen Kobalt-, Lanthaniden- und/oder Uranylionen sind.

**6.** Verfahren nach Anspruch 4, in dem die genannten Ionen Kobalt-, Dysprosium- und/oder Europiumionen sind.

**7.** Verfahren nach einem der Ansprüche 4 bis 6, in dem das Per(3,6-anhydro)-cyclodextrin-Derivat der Formel (I) entspricht, in der alle Reste $R^1$ die Gruppe $-OCH_2COOH$ darstellen und n für die Zahl 6 steht.

**8.** Verfahren nach einem der Ansprüche 4 bis 7, in dem das genannte Medium, bei dem es sich um eine wässrige Lösung handelt, das Per(3,6-anhydro)-cyclodextrin-Derivat in einem mit der wässrigen Lösung nicht mischbaren organischen Lösungsmittel gelöst ist.

**9.** Pharmazeutische Zusammensetzung zur Dekontamination eines Lebewesens an Lanthaniden und Kobalt, **dadurch gekennzeichnet, dass** sie ein Per(3, 6-anhydro)-cyclodextrin-Derivat umfasst, das einer der folgenden Formeln entspricht:

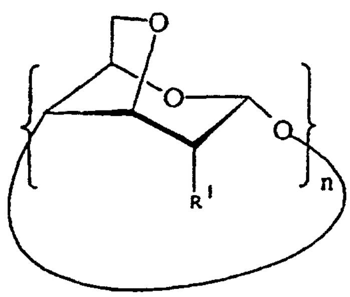
(I) und
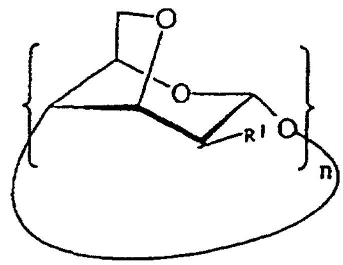
(II)

worin bedeuten: ,

mindestens einer der Reste $R^1$ die Gruppe $-OCH_2COOH$ und die übrigen Reste $R^1$, die gleich oder verschieden sein können, eine Gruppe, die einer der Formeln entspricht: OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH und $R^2$, worin $R^2$ und $R^3$, die gleich oder verschieden sein können, eine aliphatische oder aromatische, gesättigte oder ungesättigte Kohlenwasserstoffgruppe darstellen, die ein oder mehrere Heteroatome, ausgewählt aus der Gruppe O, S und N, enthalten kann, und
n die Zahl 6, 7 oder 8.

**10.** Pharmazeutische Zusammensetzung nach Anspruch 9, in der das Per(3,6-anhydro)-cyclodextrin-Derivat der Formel (I) entspricht, in der alle Reste $R^1$ die Gruppe $-OCH_2COOH$ und n die Zahl 6 darstellen.

**11.** Komplex eines Metalls, ausgewählt aus Dy, Eu, Lu, La und Co, und eines Per(3,6-anhydro)-cyclodextrin-Derivats mit einer der folgenden Formeln:

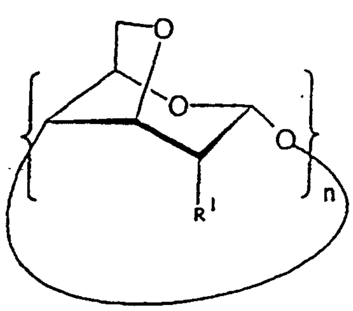
(I) und
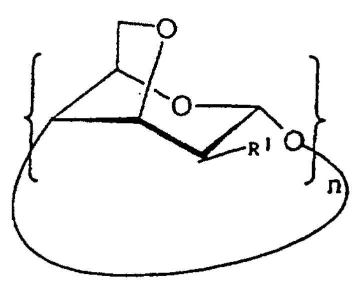
(II)

worin bedeuten:

mindestens einer der Reste $R^1$ die Gruppe $-OCH_2COOH$ und die übrigen Reste $R^1$, die gleich oder verschieden sein können, eine Gruppe, die einer der folgenden Formeln entspricht: OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH und $R^2$, worin $R^2$ und $R^3$, die gleich oder verschieden sein können, eine aliphatische oder aromatische, gesättigte oder ungesättigte Kohlenwasserstoffgruppe darstellen, die ein oder mehrere Heteroatome, ausgewählt aus der Gruppe O, S und N, enthalten kann, und
n die Zahl 6, 7 oder 8.

**12.** Komplex nach Anspruch 11, in dem das Per(3,6-anhydro)-cyclodextrin-Derivat der Formel (I) entspricht, in der alle

Reste $R^1$ die Gruppe $-OCH_2COOH$ darstellen und n für die Zahl 6 steht.

## Claims

**1.** Derivative of per - (3,6 - anhydro) - cyclodextrin that corresponds to one of the following formulae:

(I) and (II)

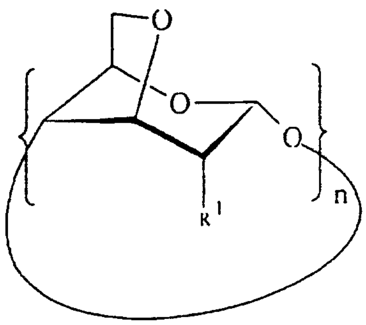

(I)

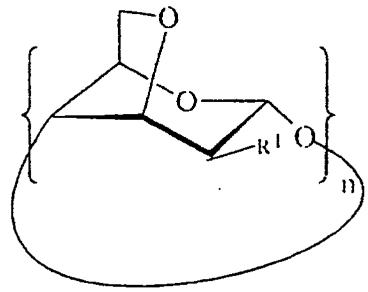

(II)

in which at least one of the $R^1$ groups represents the group $-OCH_2COOH$ and the other $R^1$ groups, which may be identical or different, represent a group that corresponds to one of the following formulae: OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH and $R^2$, in which $R^2$ and $R^3$, which may be identical or different, represent an aliphatic or aromatic hydrocarbon group, either saturated or unsaturated, which may include one or several heteroatoms comprising 0, S and N, and where n is equal to 6, 7 or 8.

**2.** Derivative of per - (3,6 - anhydro) - cyclodextrin according to claim 1, in which all of the $R^1$ groups represent the group $-OCH_2COOH$ and n is equal to 6.

**3.** Process for preparing a derivative of per - (3,6 - anhydro) - cyclodextrin that corresponds either to formula (I) or (II):

(I) and (II)

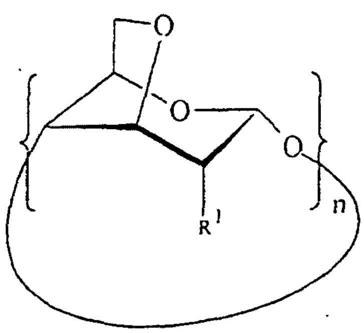

(I)

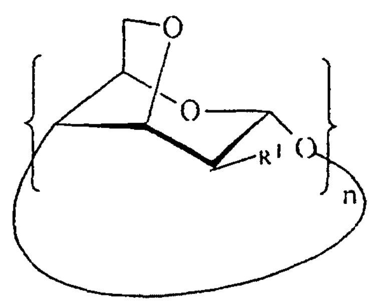

(II)

in which at least one of the $R^1$ groups represents the group $-OCH_2COOH$ and the other $R^1$ groups, which may be identical or different, represent a group that corresponds to one the following formulae: OH, $OR^2$, SH, $SR^2$, $OCOR^2$, $NH_2$, $NHR^2$, $NR^2R^3$, $CONH_2$, $CONHR^2$, $CONR^2R^3$, CN, $COOR^2$, COOH and $R^2$, in which $R^2$ and $R^3$, which may be identical or different, represent an aliphatic or aromatic hydrocarbon group, either saturated or unsaturated, which may include one or several heteroatoms comprising O, S and N, and where n is equal to 6, 7 or 8, which comprises the following stages:

- 1) a per - anhydro - cyclodextrin that corresponds to the formula:

(III) or (IV)

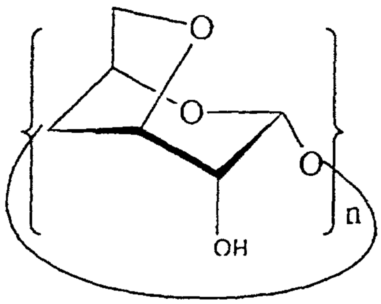 (III) ou 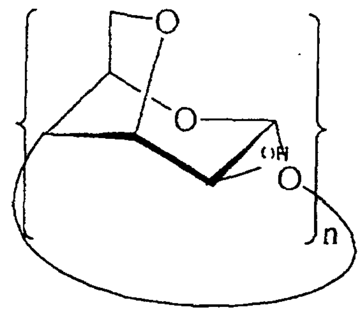 (IV)

in which n is equal to 6, 7 or 8, is reacted with an alkali metal hydride in order to convert the OH group(s) into OM group(s), where M represents an alkali metal ;

- 2) reacting, in an alkaline medium, the modified per - anhydro - cyclodextrin obtained in 1) with a halide with the formula $XCH_2COOR^4$ in which X represents a halogen atom and $R^4$ represents H, $Si(CH_3)_3$ or an alkali metal, in sufficient quantity to ensure that at least one of the OM group(s) is converted into a $CH_2COOR^4$ group ;
- 3) reacting, in the case where not all of the OM groups have been converted into $-OCH_2COOR^4$ groups, the remaining OM groups with one or several reagents in order to convert them into $R^1$ groups that are intentionally different to $-OCH_2COOH$ ; and
- 4) treating the derivative of per - anhydro - cyclodextrin obtained in 3) with an alcohol, a slightly acid medium or water in order to convert the $-OCH_2COOR^4$ groups into $-OCH_2COOH$ groups.

**4.** Process for fixing or separating ions, consisting in bringing into contact a medium containing the said ions and a derivative of per - (3,6 - anhydro) - cyclodextrin that corresponds to one of the following formulae:

(I) and (II)

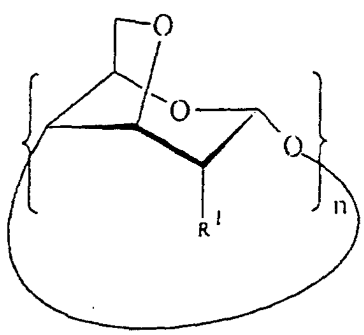 (I) 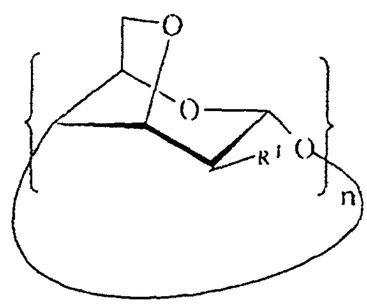 (II)

in which at least one of the $R^1$ groups represents-the group $-OCH_2COOH$ and the other $R^1$ groups, which may be identical or different, represent a group that corresponds to one of the following formulae: OH, $OR^2$, SH, $SR^2$, $OCOR^2$, $NH_2$, $NHR^2$, $NR^2R^3$, $CONHR^2$, $CONR^2R^3$, $CONH_2$, CN, $COOR^2$, COOH and $R^2$, in which $R^2$ and $R^3$, which may be identical or different, represent an aliphatic or aromatic hydrocarbon group, either saturated or unsaturated, which may include one or several heteroatoms comprising O, S and N, and n is equal to 6, 7 or 8, in order to fix the said ions in the form of a complex with the derivative of per - (3,6 - anhydro) - cyclodextrin, and separating them from the said medium.

**5.** Process according to claim 4, in which the said ions are ions of cobalt, the lanthanides and / or uranyl.

**6.** Process according to claim 4, in which the said ions are ions of cobalt, dysprosium and / or europium.

**7.** Process according to any of claims 4 to 6, in which the derivative of per - (3,6 - anhydro) - cyclodextrin corresponds to the formula (I), in which all of the $R^1$ groups represent the group $-OCH_2COOH$ and n is equal to 6.

**8.** Process according to any of claims 4 to 7, in which the said medium is an aqueous solution, and the derivative of per - (3,6 - anhydro) - cyclodextrin is dissolved in an organic solvent that is immiscible with the aqueous solution.

**9.** Pharmaceutical composition for the decontamination of lanthanides and cobalt from living organisms, whereby it comprises a derivative of per - (3,6 - anhydro) - cyclodextrin that corresponds to one of the following formulae:

(I) and (II)

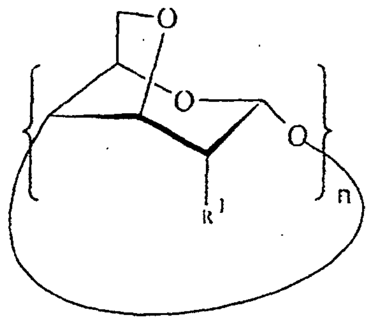

(I)

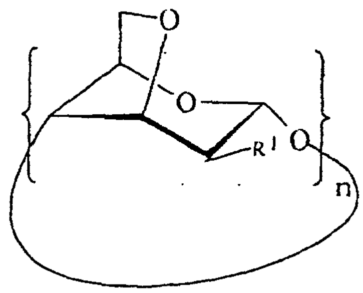

(II)

in which at least one of the $R^1$ groups represents the group $-OCH_2COOH$ and the other $R^1$ groups, which may be identical or different, represent a group that corresponds to one of the following formulae: OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH and $R^2$, in which $R^2$ and $R^3$, which may be identical or different, represent an aliphatic or aromatic hydrocarbon group, either saturated or unsaturated, which may include one or several heteroatoms comprising 0, S and N, and n is equal to 6, 7 or 8.

**10.** Pharmaceutical composition according to claim 9, in which the derivative of per - (3,6 - anhydro) - cyclodextrin corresponds to the formula (I) in which all of the $R^1$ groups represent the group $-OCH_2COOH$ and n is equal to 6.

**11.** Complex of a metal chosen from Dy, Eu, Lu, La and Co and a derivative of per - (3,6 - anhydro) - cyclodextrin that corresponds to one of the following formulae:

(I) and (II)

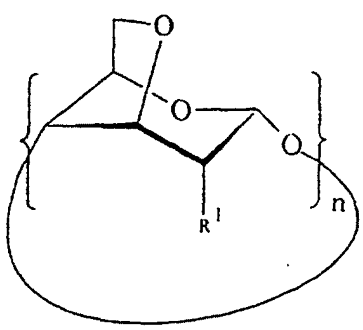

(I)

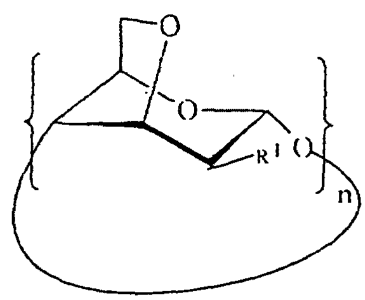

(II)

in which at least one of the $R^1$ groups represents the group $-OCH_2COOH$ and the other $R^1$ groups, which may be identical or different, represent a group that corresponds to one of the following formulae: OH, $OR^2$, SH, $SR^2$, $OCOR^2$, $NH_2$, $NHR^2$, $NR^2R^3$, $CONR^2R^3$, $CONHR^2$, $CONH_2$, CN, $COOR^2$, COOH and $R^2$, in which $R^2$ and $R^3$, which may be identical or different, represent an aliphatic or aromatic hydrocarbon group, either saturated or unsaturated, which may include one or several heteroatoms comprising O, S and N, and n is equal to 6, 7 or 8.

**12.** Complex according to claim 11,. in which the derivative of per - (3,6 - anhydro) - cyclodextrin corresponds to the formula (I) in which all of the $R^1$ groups represent the group $-OCH_2COOH$ and n is equal to 6.

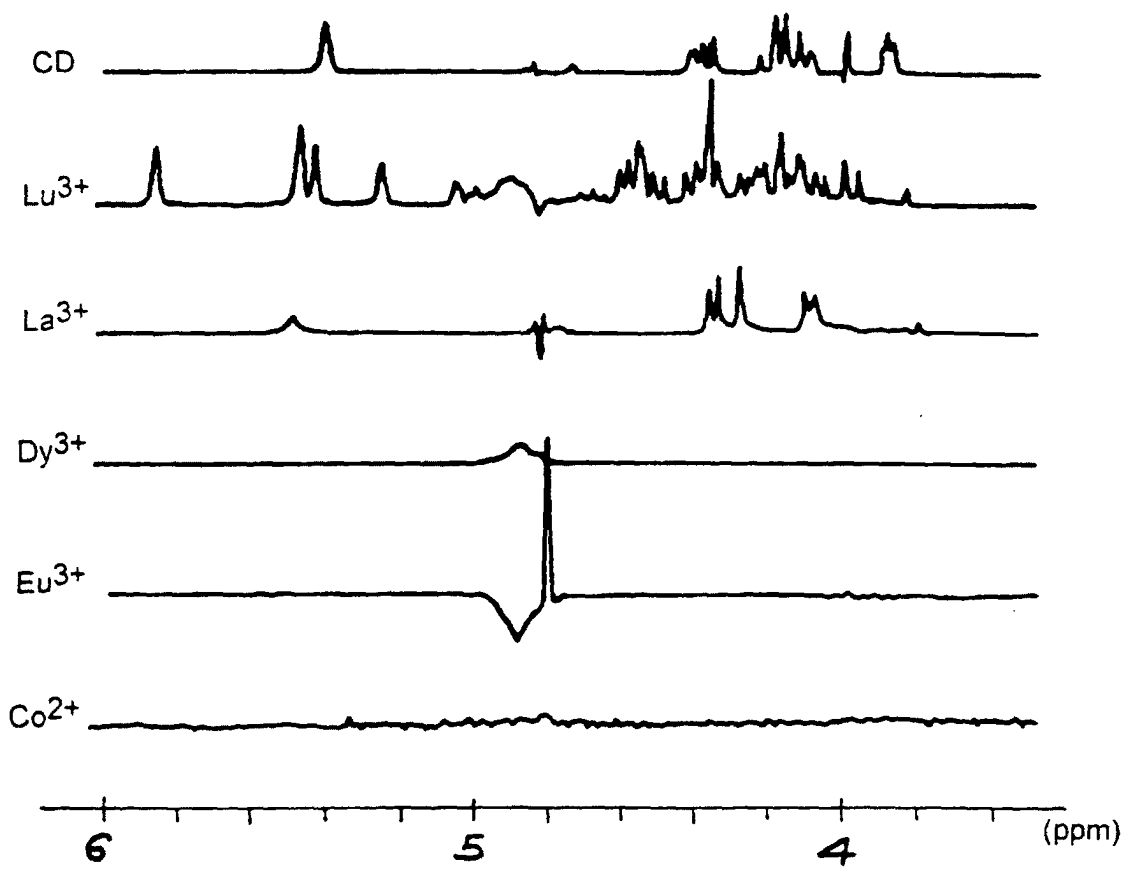


FIG. 1

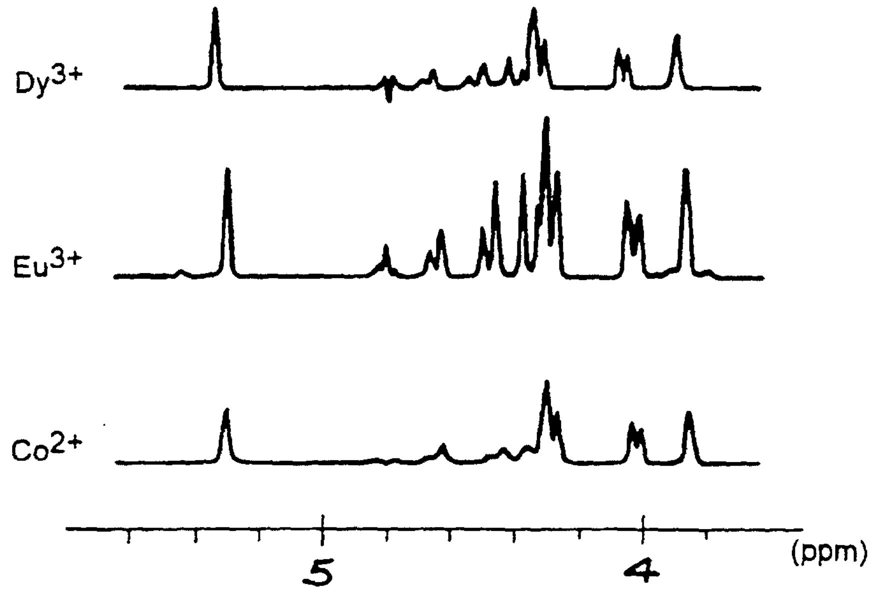


FIG. 2

FIG. 3